# EUROPEAN PATENT APPLICATION

(11) **EP 4 338 699 A1**
(43) Date of publication of application: **20.03.2024**
(21) Application number: 22195964.6
(22) Date of filing: 15.09.2022
(51) Int. Cl.: A61B 90/20, G06N 20/00, A61B 17/00, A61B 90/50

(54) **SYSTEM, METHOD, AND COMPUTER PROGRAM FOR A SURGICAL IMAGING SYSTEM**

(71) Applicant: Leica Instruments (Singapore) Pte Ltd, Singapore 608924 (SG)
(72) Inventor: ROSTYKUS, Manon, 1085 Vulliens (CH)
(74) Representative: 2SPL Patentanwälte PartG mbB

(57) **Abstract**

Examples relate to a system (110), a method, and a computer program for a surgical imaging system (100), and to a corresponding surgical imaging system comprising such a system for a surgical imaging system. The system (110) is configured to obtain an eye-tracking sensor signal from an eye-tracking system (130). The system is configured to determine a gaze of a user of a surgical imaging device (120) of the surgical imaging system on a portion of a field of view of the surgical imaging device based on the eye-tracking sensor signal. The system is configured to adjust the field of view of the surgical imaging device based on the gaze of the user on the portion of the field of view.

## Description

### Technical field

Examples relate to a system, a method, and a computer program for a surgical imaging system, and to a corresponding surgical imaging system comprising such a system for a surgical imaging system.

### Background

Surgical imaging systems are imaging systems, such as microscope systems, which are designed to be used during surgical procedures. Such imaging systems often provide a high degree of magnification. As a result, the field of view of a surgical imaging device of such a surgical imaging system is often small. During the surgical procedure, the surgeon, or an assistant, often desires to adjust the field of view, e.g., to concentrate on a different region of the surgical site the surgeon operates on.

In some cases, the surgeon may use a head-mounted display to view the surgical site, with the head-mounted display using imaging sensor data generated by the surgical imaging device. An example of such a use of a head-mounted display is given in EP 3 904 947 A1, for example.

There may be a desire for an improved concept for a surgical imaging system, which provides an improved adjustment of a field of view seen by the surgeon.

### Summary

This desire is addressed by the subject-matter of the independent claims.

Various examples of the present disclosure are based on the finding, that the field of view of a surgical imaging device, such as a microscope of a surgical microscope system, can be adjusted automatically or semi-automatically by tracking the gaze of a user of the surgical imaging device. In the proposed concept, the gaze of the user of the surgical imaging device on a portion of the field of view is determined, and the field of view is adjusted based on the determined gaze. Thus, the field of view can be adjusted without the surgeon having to perform a manual adjustment of the field of view.

Some aspects of the present disclosure relate to a system for a surgical imaging system. The system comprises one or more processors and one or more storage devices. The system is configured to obtain an eye-tracking sensor signal from an eye-tracking system. The system is configured to determine a gaze of a user of a surgical imaging device of the surgical imaging system on a portion of a field of view of the surgical imaging device based on the eye-tracking sensor signal. The system is configured to adjust the field of view of the surgical imaging device based on the gaze of the user on the portion of the field of view. By determining the field of view based on the gaze of the user, the field of view can be adjusted without the surgeon having to perform a manual adjustment of the field of view.

In some examples, the system may be configured to center the field of view around the portion of the field of view on which the gaze may be detected. Thus, the field of view may be adjusted such, that the user has an improved view of the portion of the field of view the user has gazed upon.

In some cases, a continuous adjustment of the field of view may be undesired, as the user may prefer the field of view to remain static while the surgeon performs a portion of the surgical procedure. Instead, the user may trigger the adjustment using an input device. Accordingly, the system may be configured to obtain an input signal from the input device of the surgical imaging system, and to trigger the adjustment of the field of view based on a user command for triggering the adjustment of the field of view contained in the input signal. This may avoid inadvertent adjustment of the field of view.

For example, the system may be configured to obtain the input signal from one of an input modality of a handle of the surgical imaging system, an input modality of a foot pedal of the surgical imaging system, an input modality of the surgical imaging device, a camera-based input device of the surgical imaging system, a depth-sensor based input device of the surgical imaging system and a voice-based input device of the surgical imaging system. As is evident, the surgical imaging system can contain numerous input devices, which allow providing the user command with little effort and a high amount of flexibility.

In some examples, however, the system may be configured to continuously adjust the field of view of the surgical imaging device based on the gaze of the user. In this case, no additional trigger may be necessary, which may further reduce the burden on the user. In particular, the system may be configured to continuously and gradually adjust the field of view of the surgical imaging device based on the gaze of the user. The gradual adjustment may avoid abrupt changes of the field of view, which may be disconcerting to the user.

In some examples, the adjustment of the field of view may employ image analysis techniques to identify a likely target of the gaze of the user. For example, the system may be configured to perform object-detection on imaging sensor data representing the field of view of the surgical imaging device to determine one or more regions of interest visible in the field of view. The system may be configured to adjust the field of view to a region of interest intersecting with the portion of the field of view on which the gaze may be detected. This may improve selection of the adjusted field of view, e.g., as the field of view can be adjusted such, that an improved view of the object of interest is provided to the user.

In general, the proposed concept may be used for purely optical surgical imaging devices, such as microscopes with oculars showing the optical beam path through the camera. Various examples of the present disclosure may, however, provide additional functionality if the field of view is shown as a digital view, e.g., via ocular displays, an auxiliary display (mounted at the surgical imaging system) or a head-mounted display. For example, the system may be configured to generate a digital view based on the imaging sensor data, and to highlight the region of interest intersecting with the portion of the field of view on which the gaze is detected within the field of view prior to adjusting the field of view. This may provide visual feedback to the user before the field of view is eventually adjusted based on the gaze.

Surgical imaging systems often are highly complex and versatile systems, which provide many functionalities that can be used to adjust the field of view. Accordingly, adjusting the field of view may comprise one or more of providing a control signal to a robotic adjustment system of the surgical imaging system, providing a control signal to a zoom functionality of the surgical imaging system, and adjusting a crop factor of image processing performed by the surgical imaging system (and thus applying a digital zoom). Each of the aforementioned means (robotic adjustment system, zoom functionality and image processing) has advantages, such as a high degree of freedom (robotic adjustment system) and an adjustment of the field of view without requiring movement of the surgical imaging device (zoom functionality and image processing).

After certain adjustments, such as the application of an optical zoom, the change of a working distance or a lateral movement of the surgical imaging device, the field of view may be out of focus. In some examples, the system may be configured to trigger an autofocus functionality of the surgical imaging system after adjusting the field of view of the surgical imaging device. Through the use of the autofocus functionality, the sharpness of the view on the surgical site may be ensured.

As outlined above, the proposed concept may be used with digital or hybrid surgical imaging devices, which are capable of providing a digital view on the surgical site. For example, the system may be configured to obtain imaging sensor data showing the field of view of the surgical imaging device from an optical imaging sensor of the surgical imaging device, and to generate a digital view based on the imaging sensor data, the digital view representing the adjusted field of view. This may provide additional degrees of flexibility, both with respect to the display being used (e.g., digital oculars, an auxiliary display, or a head-mounted display) and image processing being performed to allow for an augmented view on the surgical site.

In general, the gaze of the user can be determined via different modalities. For example, the system may be configured to obtain the eye-tracking sensor signal from an eye-tracking system integrated within oculars of the surgical imaging device. This option may be chosen if the display being used is an ocular display, or if the oculars provide an optical view of the surgical site (through the surgical imaging device).

Alternatively, or additionally, the system may be configured to obtain the eye-tracking sensor signal from an eye-tracking system integrated within a headset of the surgical imaging system. This option may be chosen the display being used is a head-mounted display.

An aspect of the present disclosure relates to a surgical imaging system comprising the surgical imaging device, the eye-tracking sensor and the system introduced above. For example, the surgical imaging system may be a surgical microscope system.

Some aspects of the present disclosure relate to a method for a surgical imaging system. The method comprises obtaining an eye-tracking sensor signal of an eye-tracking system. The method comprises determining a gaze of a user of a surgical imaging device of the surgical imaging system on a portion of a field of view of the surgical imaging device based on the eye-tracking sensor signal. The method comprises adjusting the field of view of the surgical imaging device based on the gaze of the user on the portion of the field of view.

An aspect of the present disclosure relates to a computer program with a program code for performing the above method when the computer program is executed on a processor.

### Short description of the Figures

Some examples of apparatuses and/or methods will be described in the following by way of example only, and with reference to the accompanying figures, in which
- Fig. 1a: shows a block diagram of an example of a system for a surgical imaging system;
- Fig. 1b: shows a schematic diagram of an example of a surgical imaging system, and in partic-ular of a surgical microscope system;
- Fig. 2: shows a flow chart of an example of a method for a surgical imaging system;
- Fig. 3: shows an example of a gaze-based adjustment of a field of view;
- Fig. 4: shows an example of a visual indicator being used to highlight a region of interest prior to adjusting a field of view; and
- Fig. 5: shows a schematic diagram of an example of a system comprising a surgical imaging device and a computer system.

### Detailed Description

Various examples will now be described more fully with reference to the accompanying drawings in which some examples are illustrated. In the figures, the thicknesses of lines, layers and/or regions may be exaggerated for clarity.

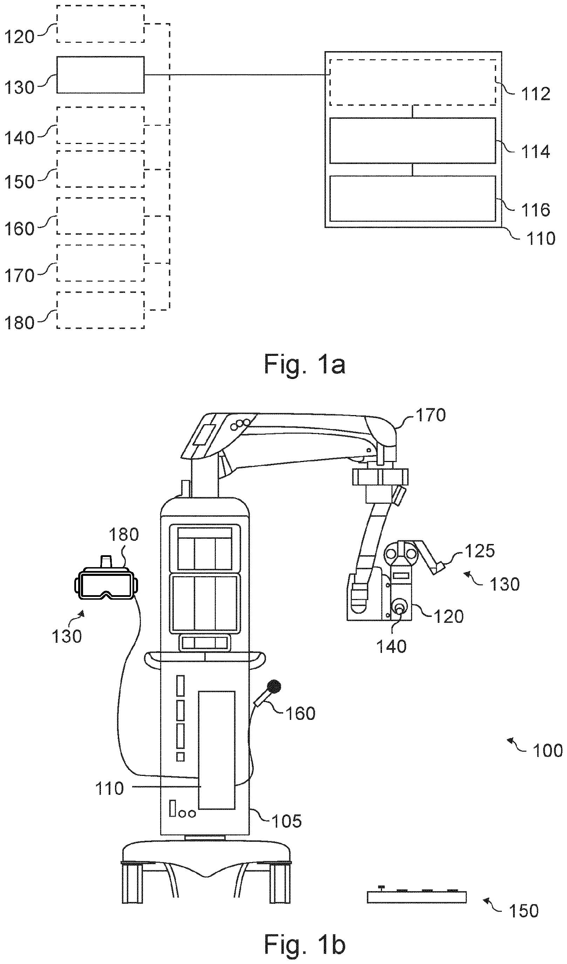

Fig. 1a shows a block diagram of an example of a system 110 for a surgical imaging system 100 (shown in more detail in Fig. 1b). The system 110 is a component of the surgical imaging system 100 and may be used to control various aspects of the surgical imaging system 100. In particular, it may be used to control (and adjust) a field of view provided by a surgical imaging device 120 of the surgical imaging system 100, through various means that will be introduced in more detail in the following. In addition, the system 110 may be configured to control additional aspects of the surgical imaging system, and/or to perform sensor data processing (e.g., imaging sensor data processing), and/or to provide a display signal for various displays of the surgical imaging system.

In general, the system 110 may be considered to be a computer system. The system 110 comprises one or more processors 114 and one or more storage devices 116. Optionally, the system 110 further comprises one or more interfaces 112. The one or more processors 114 are coupled to the one or more storage devices 116 and to the one or more interfaces 112. In general, the functionality of the system 110 may be provided by the one or more processors 114, in conjunction with the one or more interfaces 112 (for exchanging data/information with one or more other components of the surgical imaging system 100 (and outside the surgical imaging system 100), such as an optical imaging sensor of the surgical imaging device 120, an eye-tracking system 130, an input device 140; 150; 160 of the surgical imaging system, a robotic adjustment system 170 and/or a headset/head-mounted display 180), and with the one or more storage devices 116 (for storing information, such as machine-readable instructions of a computer program being executed by the one or more processors). In general, the functionality of the one or more processors 114 may be implemented by one or more processors 114 executing machine-readable instructions. Accordingly, any feature ascribed to the one or more processors 114 may be defined by one or more instructions of a plurality of machine-readable instructions. The system 110 may comprise the machine-readable instructions, e.g., within the one or more storage devices 116.

As outlined above, the system 110 is part of the surgical imaging system 100, which comprises various components in addition to the system 110. For example, the surgical imaging system 100 comprises the surgical imaging device 120, and may comprise one or more additional components, such as the aforementioned eye-tracking system 130, input device 140; 150; 160, robotic adjustment system 170 and/or a headset/head-mounted display 180. Fig. 1b shows a schematic diagram of an example of such a surgical imaging system 100, and in particular of a surgical microscope system 100. In the following, the surgical imaging system 100 may also be referred to as surgical microscope system 100, which is a surgical imaging system 100 that comprises a (surgical) microscope as surgical imaging device 120. However, the proposed concept is not limited to such embodiments. The surgical imaging system 100 may be based on various (single or multiple) surgical imaging devices, such as one or more microscopes, one or more endoscopes, one or more radiation imaging devices, one or more surgical tomography devices (such as optical coherence tomography devices) etc. Accordingly, the surgical imaging system may alternatively be a surgical endoscope system, a surgical radiation imaging system or a surgical tomography system. However, the following illustrations assume that the surgical imaging device 120 is a (surgical) microscope, and that the surgical imaging system 100 is a surgical microscope system 100.

Accordingly, the surgical imaging system or surgical microscope system 100 may comprise a microscope 120. In general, a microscope, such as the microscope 120, is an optical instrument that is suitable for examining objects that are too small to be examined by the human eye (alone). For example, a microscope may provide an optical magnification of a sample. The microscope may be a purely optical microscope (where the oculars show the light travelling from the surgical site being observed through the microscope 120), a hybrid (optical and digital) microscope, or a digital microscope (where the oculars or a display show a digital view of the surgical site). In modern hybrid or digital microscopes, the optical magnification is (also) provided for an optical imaging sensor. In the latter two cases, the microscope 120 thus comprises an optical imaging sensor, which is coupled with the system 110. The microscope 120 may further comprise one or more optical magnification components that are used to magnify a view on the sample, such as an objective (i.e., lens). For example, the surgical imaging device or microscope 120 is often also called the "optics carrier" of the surgical imaging system.

There are a variety of different types of surgical imaging devices. If the surgical imaging device is used in the medical or biological fields, the object being viewed through the surgical imaging device may be a sample of organic tissue, e.g., arranged within a petri dish or present in a part of a body of a patient. In various examples presented here, the surgical imaging device 120 may be a microscope of a surgical microscope system, i.e., a microscope that is to be used during a surgical procedure, such as an oncological surgical procedure or during tumor surgery. Accordingly, the object being viewed through the surgical imaging device (that is shown in the field of view of the surgical imaging device) and shown in the digital view generated by based on the imaging sensor data provided by the (optional) optical imaging sensor, may be a sample of organic tissue of a patient, and may be in particular be the surgical site that the surgeon operates on during the surgical procedure. For example, the object to be imaged, i.e., the surgical site, may be a surgical site of a brain during the course of neurosurgery. However, the proposed concept is also suitable for other types of surgery, such as cardiac surgery or ophthalmology.

In general, a surgical imaging system, such as the surgical microscope system 100, is a system that comprises a microscope 120 and additional components, which are operated together with the microscope, such as the system 110 (which may be a computer system being adapted to control the surgical microscope system, and, for example, process imaging sensor data of the microscope), and additional sensors, displays etc.

Fig. 1b shows a schematic diagram of an example of a surgical imaging system 100, and in particular of a surgical microscope system 100, comprising the system 110 and a microscope 120. The surgical microscope system 100 shown in Fig. 1b comprises a number of optional components, such as a base unit 105 (comprising the system 110) with a (rolling) stand, oculars 125 (e.g., with ocular displays) that are arranged at the microscope 120, the eye-tracking system 130, an auxiliary display that is arranged at the base unit 105, the one or more input devices 140; 150; 160 and the robotic adjustment system 170, which is shown as a (robotic or manual) arm 170 which holds the microscope 120 in place, and which is coupled to the base unit 105 and to the microscope 120. In general, these optional and non-optional components may be coupled to the system 110 which may be configured to control and/or interact with the respective components. In Fig. 1b, the connection between the headset 180 and the system 110, and the connection between two examples of eye-tracking system 130 (as part of the headset 180, or as part of the oculars 125) are shown as wired connections. In other words, the headset 180 and/or the eye-tracking system 130 may be connected to the system 110 using a wired connection, i.e., they may be wire bound devices. Accordingly, the interface 112 may be configured to communicate with the headset 180 and/or the eye-tracking system 130 using a wired connection. However, the proposed concept is not limited to these examples. Alternatively, the headset 180 and/or the eye-tracking system may be wireless devices, i.e., a wireless headset 180 comprising the eye-tracking system 130, i.e., they may be connected to the system 110 using a wireless (data) connection. Accordingly, the interface 112 may be configured to communicate with the headset 180 and/or the eye-tracking system 130 using a wireless (data) connection.

In the proposed concept, the system 110 is, in particular, used to control the field of view of the surgical imaging device 120. This is done by tracking the gaze of a user (e.g., the surgeon or an assistant responsible for controlling the surgical imaging system), and by adjusting the field of view accordingly. For example, the system 110 is configured to obtain an eye-tracking sensor signal from the eye-tracking system 130. The system 110 is configured to determine a gaze of a user of a surgical imaging device 120 of the surgical imaging system on a portion of a field of view of the surgical imaging device based on the eye-tracking sensor signal. The system 110 is configured to adjust the field of view of the surgical imaging device based on the gaze of the user on the portion of the field of view.

To perform the gaze-based adjustment of the field of view, the system uses an eye-tracking sensor signal from the eye-tracking system 130. In general, the eye-tracking sensor signal may be indicative of the gaze of the user, i.e., of the direction of view of the user. For example, eye-tracking sensor signal may indicate where the user is looking, e.g., a portion of a display, or a center or peripheral region of an ocular. The implementation of the eye-tracking sensor signal may depend on the eye-tracking sensor being used. For example, the system may be configured to obtain the eye-tracking sensor signal from an eye-tracking system integrated within oculars 125 of the surgical imaging device. In this case, the eye-tracking sensor signal may indicate whether the user is looking towards a central portion of the field of view provided by the oculars, or towards a peripheral portion of the field of view provided by the oculars (and the direction thereof, e.g., to the right, left, top, bottom, top-right, bottom-left etc.). Alternatively, the system 110 may be configured to obtain the eye-tracking sensor signal from an eye-tracking sensor that is coupled with a display, e.g., with the aforementioned auxiliary display, or that is part of the headset/head-mounted display. For example, the system may be configured to obtain the eye-tracking sensor signal from an eye-tracking system integrated within a headset 180 of the surgical imaging system. In these cases, the eye-tracking sensor signal may indicate a portion of the display that the user is looking towards (e.g., focusing on).

The system then uses the eye-tracking sensor signal to determine the gaze of the user, and in particular the portion of the field of view the gaze is directed at. For example, the system may be configured to, if the eye-tracking sensor signal indicates the direction of view of the user (e.g., towards the center or towards the periphery (and direction thereof)), estimate the portion of the field of vie the gaze is directed at from the direction of view. Alternatively, the system may be configured to, if the eye-tracking sensor signal indicates a portion of a display the user is looking towards (e.g., within the head-mounted display or on the aforementioned auxiliary display), translate the portion of the display to a corresponding portion of the field of view. In both cases, the system determines a portion of the field of view currently provided to the user that the user is looking towards. In the present context, the field of view of the surgical imaging device is the field of view provided by the surgical imaging device to the user, e.g., the field of view the user sees when the user looks through the oculars or looks at a display of the surgical imaging device. In some examples, when multiple displays are used (e.g., multiple head-mounted displays and/or multiple digital oculars), the field of view may differ for different users of the surgical imaging system, e.g., through the use of user-specific image cropping and/or the application of a user-specific digital zoom.

In the previous examples, the portion of the field of view was used without reference to specific features of interest. Instead, merely a portion of the field of view was referenced. In some cases, however, additional image processing may be used to identify one or more specific regions of interest (e.g., anatomical features of interest, or, more generally, objects of interest (such as surgical markers etc.) within the field of view, and to take them into account when adjusting the field of view of the surgical imaging device. For example, the system may be configured to perform (machine-learning based) object-detection (or, more generally, image segmentation) on imaging sensor data representing the field of view of the surgical imaging device to determine one or more regions of interest (e.g., anatomical, or non-anatomical features or objects of interest) visible in the field of view. For example, the imaging sensor data may be analyzed to determine and distinguish features in the imaging sensor data, such as anatomical features (e.g., blood vessels, tumors, branches, portions of tissue etc.) or non-anatomical features (e.g., markers, clips, or stitches). For this, one or both of the following machine-learning-based techniques may be used - image segmentation and object detection. The system may be configured to perform image segmentation and/or object detection to determine the features, and thus the regions of interest, within the imaging sensor data. In object detection, the location of one or more pre-defined objects (i.e., objects that the respective machine-learning model is trained on) in the imaging sensor data is output by a machine-learning model, along with a classification of the object (if the machine-learning model is trained to detect multiple different types of objects). In general, the location of the one or more pre-defined objects is provided as a bounding box, i.e., a set of positions forming a rectangular shape that surrounds the respective object being detected. In image segmentation, the location of features (i.e., portions of the imaging sensor data that have similar attributes, e.g., that belong to the same object) are output by a machine-learning model. In general, the location of the features is provided as a pixel mask, i.e., the location of pixels that belong to a feature are output on a per-feature basis.

For both object detection and image segmentation, a machine-learning model is used that is trained to perform the respective task. For example, to train the machine-learning model being trained to perform object detection, a plurality or samples of imaging sensor data may be provided as training input samples, and a corresponding listing of bounding box coordinates may be provided as desired output of the training, with a supervised learning-based training algorithm being used to perform the training using the plurality of training input samples and corresponding desired output. For example, to train the machine-learning model being trained to perform image segmentation, the plurality or samples of imaging sensor data may be provided as training input samples, and corresponding pixel masks may be provided as desired output of the training, with a supervised learning-based training algorithm being used to perform the training using the plurality of training input samples and corresponding desired output. In some examples, the same machine-learning model may be used to perform both object detection and image segmentation. In this case, the two above-mentioned types of desired output may be used in parallel during the training, with the machine-learning model being trained to output both the bounding boxes and the pixel masks. The machine-learning model may thus be used to determine one or more potential features of interest, and thus the one or more regions of interest.

These one or more regions of interest may now be used to support the determination of the portion of the field of view on which the gaze is detected. For example, the system may be configured to correlate the gaze of the user with the one or more (potential) regions of interest and determine the portion of the field of view such, that it intersects with one of the one or more regions of interest. In effect, the system may be configured to adjust the field of view to a region of interest intersecting with the portion of the field of view on which the gaze is detected.

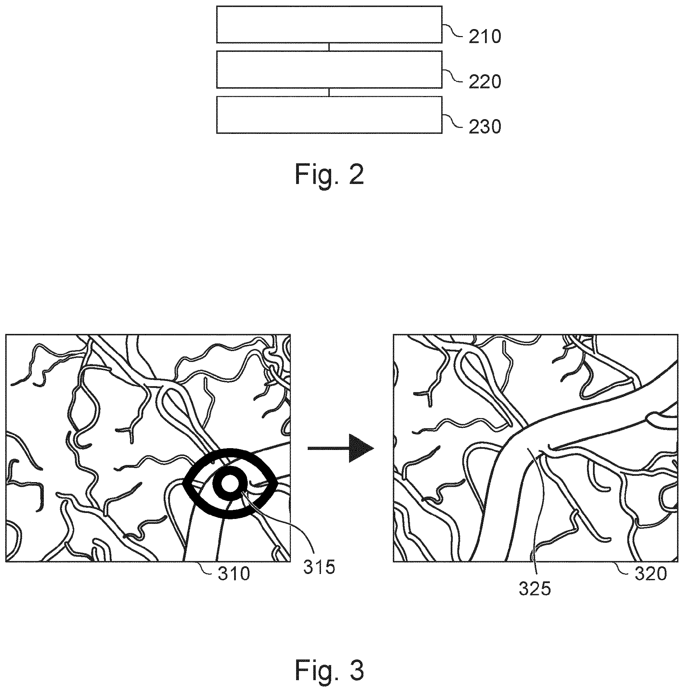

The system is then configured to adjust the field of view of the surgical imaging device based on the gaze of the user on the portion of the field of view. For example, the system may be configured to adjust the field of view based on the portion of the field of view the gaze is directed towards. Since the user is looking towards this portion of the field of view, the field of view may be adjusted (e.g., moved) such, that the portion of the field of view the gaze is directed towards moves towards the center of the field of view. An example of such an adjustment is shown in Fig. 3, for example. The system may be configured to center the field of view around the portion of the field of view on which the gaze is detected. In other words, the field of view may be adjusted such, that the portion of the field of view on which the gaze is detected prior to the adjustment, is at the center (or closer to the center) of the field of view after the adjustment.

In general, there are different means for adjusting the field of view. In some cases, the entire surgical imaging device may be moved (e.g., laterally, or vertically), which affects the field of view. Accordingly, adjusting the field of view may comprise providing a control signal to a robotic adjustment system 170 (e.g., a robotic arm) of the surgical imaging system (e.g., via the one or more interfaces), with the robotic adjustment system 170 being configured to move the surgical imaging device relative to the surgical site based on the control signal. Additionally, or alternatively, an optical and/or digital zoom functionality of the surgical imaging device (or of the system) may be used to increase or decrease the magnification, which also affects the field of view. Thus, adjusting the field of view may comprise providing a control signal to a zoom functionality of the surgical imaging system (e.g., via the one or more interfaces 112). Finally, a crop factor of the field of view shown in the digital view may be adjusted, which may act as a digital zoom and/or as a means to show an off-center portion of the field of view captured by the optical imaging sensor of the surgical imaging device. Accordingly, adjusting the field of view may comprise adjusting a crop factor of image processing performed by the surgical imaging system (e.g., by the system 110), e.g., as part of the generation of the digital view. After the field of view is adjusted (in particular, when the robotic adjustment system is used), the focus of the surgical imaging device may be readjusted to improve the image quality after the adjustment of the field of view. For example, the system may be configured to trigger an autofocus functionality of the surgical imaging system after adjusting the field of view of the surgical imaging device.

The proposed concept provides a convenient and intuitive means for adjusting the field of view to a portion that is of interest of the user. However, some things are only temporary interest to a surgeon. Moreover, in an hour-long surgical procedure, the surgeon may need to take their eyes off the main focus of the surgical procedure once in a while. In such situations, an adjustment of the field of view may be undesired. Therefore, the adjustment of the field of view may be triggered, or confirmed, via an input device of the surgical imaging system. In various examples, the system may be configured to obtain an input signal from an input device 140, 150, 160, of the surgical imaging system, and to trigger the adjustment of the field of view based on a user command for triggering the adjustment of the field of view contained in the input signal. For example, in some examples, the system may be configured to detect the gaze of the user to be outside a center portion of the field of view and prompt the user to confirm that an adjustment of the field of view is desired, with the user command being a confirmation after (e.g., in response to the prompt). Alternatively, the system may be configured to initiate the determination of the gaze of the user based on the user command, and then perform the adjustment after the user-triggered determination of the gaze of the user. As surgical imaging systems are often complex systems with a myriad of input devices, various of these devices may be used to provide the input signal. For example, the system may be configured to obtain the input signal from one of an input modality (e.g., a button, knob, control stick or touch surface) of a handle 140 of the surgical imaging system, an input modality (e.g., a button or control stick) of a foot pedal 150 of the surgical imaging system, an input modality of the surgical imaging device (e.g., a button, knob, touch surface or touch screen arranged at the surgical imaging device), a camera-based input device of the surgical imaging system (e.g., imaging sensor data of a user gesture), a depth-sensor based input device of the surgical imaging system (e.g., depth sensor data representing a user gesture) and a voice-based input device 160 (e.g., audio data comprising a spoken version of the user command) of the surgical imaging system. The system may be configured to process the respective input data to determine (e.g., extract, derive) the user command for triggering the adjustment of the field of view from the input signal.

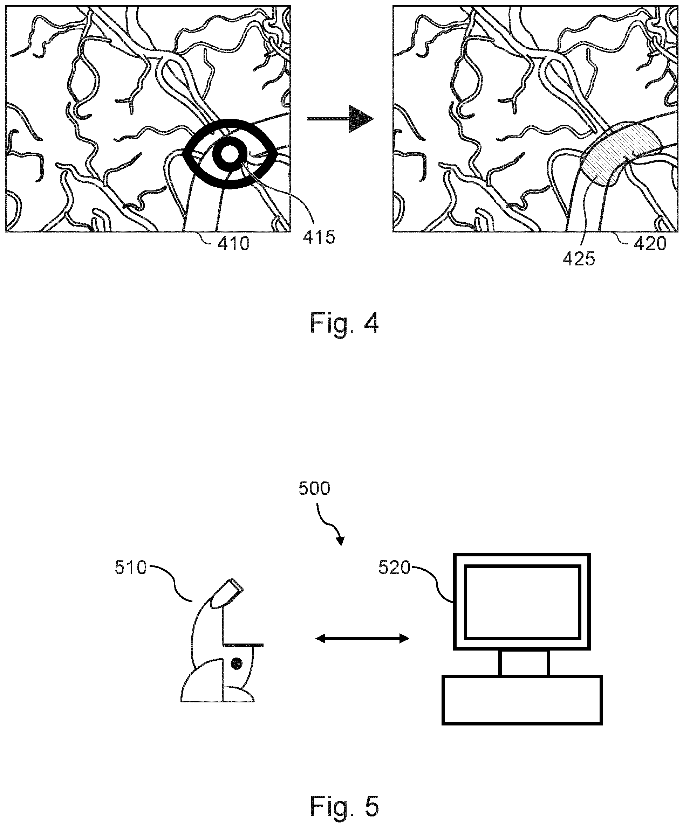

To support the adjustment of the field of view, the system may provide visual markers that indicate that an adjustment is about to take place, and a target (e.g., central area) of the adjusted field of view (which may usually correspond to the portion of the field of view the gaze is directed at. As outlined above, the system may be configured to generate a digital view based on the imaging sensor data. The system may be configured to highlight the portion of the field of view the gaze is directed towards, and therefore the central area of the adjusted field of view, in the digital view prior to adjusting the field of view. For example, the region of interest intersecting with the portion of the field of view may be highlighted. In other words, the system may be configured to highlight the region of interest intersecting with the portion of the field of view on which the gaze is detected within the field of view prior to adjusting the field of view. An example of such a user of a visual marker to highlight the region of interest is shown in Fig. 4. After being shown the highlighted portion of the digital view, the user may confirm the adjustment via the input signal. In other words, the system may be configured to trigger the adjustment of the field of view after the user has been shown the digital view with the highlighted portion and the input signal comprises a user command indicating a confirmation of the adjusted field of view.

In some other examples, the adjustment may be performed without additional trigger or confirmation by the user. For example, the system may be configured to continuously (and automatically, i.e., without user input) adjust the field of view of the surgical imaging device based on the gaze of the user. To avoid abrupt and unintended changes of the field of view, the adjustment may be limited with respect to extent and abruptness. For example, the system may be configured to continuously and gradually adjust the field of view of the surgical imaging device based on the gaze of the user, e.g., by limiting a maximal change of the field of view (e.g., in millimeters) per unit of time.

In various examples, a digital view of the surgical site is created and provided to a display of the surgical imaging system as part of a display signal. To generate the digital view, as mentioned before, the system may be configured to obtain imaging sensor data showing the field of view of the surgical imaging device from an optical imaging sensor of the surgical imaging device, and to generate a digital view based on the imaging sensor data. In the proposed system, the digital view may represent the adjusted field of view. The digital view may be viewed by the user, e.g., the surgeon, of the surgical imaging system. For this purpose, the display signal may be provided to the display, e.g., an auxiliary display arranged at the base unit 105 of the surgical microscope system, ocular displays integrated within the oculars 125 of the surgical imaging device, or one or two displays of the aforementioned headset / heat-mounted display 180. Accordingly, the system may be configured to generate the display signal for the display device of the microscope system, with the display signal being based on the digital view. For example, the display signal may be a signal for driving (e.g., controlling) the respective display device. For example, the display signal may comprise video data and/or control instructions for driving the display. For example, the display signal may be provided via one of the one or more interfaces 112 of the system. Accordingly, the system 110 may comprise a video interface 112 that is suitable for providing the display signal to the display 13 0 of the microscope system 100.

In the proposed surgical imaging system, at least one optical imaging sensor may be used to provide the aforementioned imaging sensor data. Accordingly, the optical imaging sensor, which may be part of the surgical imaging device 120 (e.g., of the microscope) may be configured to generate the imaging sensor data. For example, the at least one optical imaging sensor of the surgical imaging device 120 may comprise or be an APS (Active Pixel Sensor) - or a CCD (Charge-Coupled-Device)-based imaging sensor. For example, in APS-based imaging sensors, light is recorded at each pixel using a photodetector and an active amplifier of the pixel. APS-based imaging sensors are often based on CMOS (Complementary Metal-Oxide-Semiconductor) or S-CMOS (Scientific CMOS) technology. In CCD-based imaging sensors, incoming photons are converted into electron charges at a semiconductor-oxide interface, which are subsequently moved between capacitive bins in the imaging sensors by a circuitry of the imaging sensors to perform the imaging. The system 110 may be configured to obtain (i.e., receive or read out) the imaging sensor data from the optical imaging sensor. The imaging sensor data may be obtained by receiving the imaging sensor data from the optical imaging sensor (e.g., via the interface 112), by reading the imaging sensor data out from a memory of the optical imaging sensor (e.g., via the interface 112), or by reading the imaging sensor data from a storage device 116 of the system 110, e.g., after the imaging sensor data has been written to the storage device 116 by the optical imaging sensor or by another system or processor.

The one or more interfaces 112 of the system 110 may correspond to one or more inputs and/or outputs for receiving and/or transmitting information, which may be in digital (bit) values according to a specified code, within a module, between modules or between modules of different entities. For example, the one or more interfaces 112 may comprise interface circuitry configured to receive and/or transmit information. The one or more processors 114 of the system 110 may be implemented using one or more processing units, one or more processing devices, any means for processing, such as a processor, a computer or a programmable hardware component being operable with accordingly adapted software. In other words, the described function of the one or more processors 114 may as well be implemented in software, which is then executed on one or more programmable hardware components. Such hardware components may comprise a general-purpose processor, a Digital Signal Processor (DSP), a micro-controller, etc. The one or more storage devices 116 of the system 110 may comprise at least one element of the group of a computer readable storage medium, such as a magnetic or optical storage medium, e.g., a hard disk drive, a flash memory, Floppy-Disk, Random Access Memory (RAM), Programmable Read Only Memory (PROM), Erasable Programmable Read Only Memory (EPROM), an Electronically Erasable Programmable Read Only Memory (EEPROM), or a network storage.

More details and aspects of the system and surgical imaging system are mentioned in connection with the proposed concept, or one or more examples described above or below (e.g., Fig. 2 to 5). The system and/or surgical microscope system may comprise one or more additional optional features corresponding to one or more aspects of the proposed concept, or one or more examples described above or below.

Fig. 2 shows a flow chart of an example of a corresponding method for a surgical imaging system, e.g., for the surgical imaging system 100 shown in connection with Figs. 1a and/or 1. The method comprises obtaining 210 an eye-tracking sensor signal of an eye-tracking system. The method comprises determining 220 a gaze of a user of a surgical imaging device of the surgical imaging system on a portion of a field of view of the surgical imaging device based on the eye-tracking sensor signal. The method comprises adjusting 230 the field of view of the surgical imaging device based on the gaze of the user on the portion of the field of view.

For example, the method may be performed by the system and/or surgical imaging system introduced in connection with one of the Figs. 1a to 1b. Features introduced in connection with the system and surgical imaging system of Figs. 1a to 1b may likewise be included in the corresponding method.

More details and aspects of the method are mentioned in connection with the proposed concept, or one or more examples described above or below (e.g., Fig. 1a to 1b, 3 to 5). The method may comprise one or more additional optional features corresponding to one or more aspects of the proposed concept, or one or more examples described above or below.

In the following, two examples of the proposed concept are shown. Fig. 3 shows an example of a gaze-based adjustment of a field of view. In Fig. 3, the field of view provided by the surgical imaging device is shown at two points in time - a first field of view 310 before the adjustment, and a second field of view 320 after the adjustment. Overlaid over the first field of view 310, the target 315 of the gaze of the user/surgeon is shown overlaid over the field of view (visualized by the "eye" symbol). After adjustment of the field of view, the adjusted field of view 320 is centered around the previous target 325 of the gaze of the user.

In Fig. 4, an example is shown, where the adjustment of the field of view is visualized. Fig. 4 shows an example of a visual indicator 425 being used to highlight a region of interest prior to adjusting a field of view. Similar to Fig. 3, on the left, a first field of view 410 is shown, with a target 415 of the gaze of the user. On the right, the same field of view 420 is shown, with a visual marker 425 highlighting the region of interest intersecting with the target 415.

Various examples of the present disclosure relate to a digital apparatus to control a visualization of a region of interest (i.e., to a ROI finder). The proposed ROI finder may be used to change the region of interest displayed in, for example, but not limited to, a digital viewer.

In other systems, the user often has to manually and/or deliberately move the microscope to change the ROI the user or surgeon sees. In contrast, in the proposed concept, the region visualized with the surgical imaging device (e.g., microscope) via a display (digital viewer, screen etc.) may be changed automatically according to where the user/surgeon is looking in the image (i.e., current field of view).

The proposed concept may result in gain of time for the user/surgeon and/or an increase safety for the patient, since the user/surgeon might not need to touch the surgical imaging device/microscope to see the ROI the user/surgeon wants to see (if the surgeon usually uses handles to change the ROI). If the surgeon usually uses a mouthpiece of the surgical imaging system to change the ROI, the proposed concept may result in an improved concept for the surgeon, as the surgeon might not need to use the mouthpiece (or might use it only to confirm or trigger the adjustment of the field of view) to adjust the field of view to see the ROI the surgeon/user wants to see.

More details and aspects of the ROI finder are mentioned in connection with the proposed concept, or one or more examples described above or below (e.g., Fig. 1a to 2, 5). The ROI finder may comprise one or more additional optional features corresponding to one or more aspects of the proposed concept, or one or more examples described above or below.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some embodiments relate to a microscope comprising a system as described in connection with one or more of the Figs. 1 to 4. Alternatively, a microscope may be part of or connected to a system as described in connection with one or more of the Figs. 1 to 4. Fig. 5 shows a schematic illustration of a system 500 configured to perform a method described herein. The system 500 comprises a microscope 510 and a computer system 520. The microscope 510 is configured to take images and is connected to the computer system 520. The computer system 520 is configured to execute at least a part of a method described herein. The computer system 520 may be configured to execute a machine learning algorithm. The computer system 520 and microscope 510 may be separate entities but can also be integrated together in one common housing. The computer system 520 may be part of a central processing system of the microscope 510 and/or the computer system 520 may be part of a subcomponent of the microscope 510, such as a sensor, an actor, a camera or an illumination unit, etc. of the microscope 510.

The computer system 520 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 520 may comprise any circuit or combination of circuits. In one embodiment, the computer system 520 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 520 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 520 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 520 may also include a display device, one or more speakers, and a keyboard and/or controller, which can include a mouse, trackball, touch screen, voice-recognition device, or any other device that permits a system user to input information into and receive information from the computer system 520.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a nontransitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

Embodiments may be based on using a machine-learning model or machine-learning algorithm. Machine learning may refer to algorithms and statistical models that computer systems may use to perform a specific task without using explicit instructions, instead relying on models and inference. For example, in machine-learning, instead of a rule-based transformation of data, a transformation of data may be used, that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output. By training the machine-learning model with a large number of training images and/or training sequences (e.g. words or sentences) and associated training content information (e.g. labels or annotations), the machine-learning model "learns" to recognize the content of the images, so the content of images that are not included in the training data can be recognized using the machine-learning model. The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model. The provided data (e.g. sensor data, meta data and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e. each training sample is associated with a desired output value. By specifying both training samples and desired output values, the machine-learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g. a classification algorithm, a regression algorithm or a similarity learning algorithm. Classification algorithms may be used when the outputs are restricted to a limited set of values (categorical variables), i.e. the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine-learning model. In unsupervised learning, (only) input data might be supplied and an unsupervised learning algorithm may be used to find structure in the input data (e.g. by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e. outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g. a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g. be used to store, manipulate or apply the knowledge.

Machine-learning algorithms are usually based on a machine-learning model. In other words, the term "machine-learning algorithm" may denote a set of instructions that may be used to create, train or use a machine-learning model. The term "machine-learning model" may denote a data structure and/or set of rules that represents the learned knowledge (e.g. based on the training performed by the machine-learning algorithm). In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g. of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e. to achieve a desired output for a given input.

Alternatively, the machine-learning model may be a support vector machine, a random forest model or a gradient boosting model. Support vector machines (i.e. support vector networks) are supervised learning models with associated learning algorithms that may be used to analyze data (e.g. in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection.

### List of reference Signs

- 100: Surgical imaging system / surgical microscope system
- 110: System
- 112: Interface
- 114: Processor
- 116: Storage device
- 120: Surgical imaging device / microscope
- 125: Oculars
- 130: Eye-tracking system
- 140: Handle
- 150: Foot pedal
- 160: Voice-based input device
- 170: Robotic arm
- 180: Headset / head-mounted display
- 210: Obtaining an eye-tracking sensor signal
- 220: Determining a gaze of a user
- 230: Adjusting a field of view
- 310: First field of view
- 315: Target
- 320: Second field of view
- 325: Previous target
- 410: Field of view
- 415: Target
- 420: Field of view
- 425: Visual marker
- 500: System
- 510: Microscope
- 520: Computer system

## Claims

1. A system (110; 520) for a surgical imaging system (100; 500), the system comprising one or more processors (114) and one or more storage devices (116), wherein the system is configured to:
obtain an eye-tracking sensor signal from an eye-tracking system (130);
determine a gaze of a user of a surgical imaging device (120; 510) of the surgical imaging system on a portion of a field of view of the surgical imaging device based on the eye-tracking sensor signal; and
adjust the field of view of the surgical imaging device based on the gaze of the user on the portion of the field of view.

2. The system according to claim 1, wherein the system is configured to center the field of view around the portion of the field of view on which the gaze is detected.

3. The system according to one of the claims 1 or 2, wherein the system is configured to obtain an input signal from an input device (140; 150) of the surgical imaging system, and to trigger the adjustment of the field of view based on a user command for triggering the adjustment of the field of view contained in the input signal.

4. The system according to claim 3, wherein the system is configured to obtain the input signal from one of an input modality of a handle (140) of the surgical imaging system, an input modality of a foot pedal (150) of the surgical imaging system, an input modality of the surgical imaging device, a camera-based input device of the surgical imaging system, a depth-sensor based input device of the surgical imaging system and a voice-based input device (160) of the surgical imaging system.

5. The system according to one of the claims 1 or 2, wherein the system is configured to continuously adjust the field of view of the surgical imaging device based on the gaze of the user.

6. The system according to claim 5, wherein the system is configured to continuously and gradually adjust the field of view of the surgical imaging device based on the gaze of the user.

7. The system according to one of the claims 1 to 6, wherein the system is configured to perform object-detection on imaging sensor data representing the field of view of the surgical imaging device to determine one or more regions of interest visible in the field of view, and to adjust the field of view to a region of interest intersecting with the portion of the field of view on which the gaze is detected.

8. The system according to claim 7, wherein the system is configured to generate a digital view based on the imaging sensor data, and to highlight the region of interest intersecting with the portion of the field of view on which the gaze is detected within the field of view prior to adjusting the field of view.

9. The system according to one of the claims 1 to 8, wherein adjusting the field of view comprises one or more of providing a control signal to a robotic adjustment system (170) of the surgical imaging system, providing a control signal to a zoom functionality of the surgical imaging system, and adjusting a crop factor of image processing performed by the surgical imaging system.

10. The system according to one of the claims 1 to 9, wherein the system is configured to obtain imaging sensor data showing the field of view of the surgical imaging device from an optical imaging sensor of the surgical imaging device, and to generate a digital view based on the imaging sensor data, the digital view representing the adjusted field of view.

11. The system according to one of the claims 1 to 10, wherein the system is configured to obtain the eye-tracking sensor signal from an eye-tracking system integrated within oculars (125) of the surgical imaging device, or wherein the system is configured to obtain the eye-tracking sensor signal from an eye-tracking system integrated within a headset (180) of the surgical imaging system.

12. A surgical imaging system (100; 500) comprising a surgical imaging device (120; 510), an eye-tracking sensor and the system (110; 520) according to one of the claims 1 to 11.

13. The surgical imaging system according to claim 12, wherein the surgical imaging system is a surgical microscope system.

14. A method for a surgical imaging system, the method comprising:
Obtaining (210) an eye-tracking sensor signal of an eye-tracking system;
Determining (220) a gaze of a user of a surgical imaging device of the surgical imaging system on a portion of a field of view of the surgical imaging device based on the eye-tracking sensor signal; and
adjusting (230) the field of view of the surgical imaging device based on the gaze of the user on the portion of the field of view.

15. A computer program with a program code for performing the method according to claim 14 when the computer program is executed on a processor.
